# EUROPÄISCHE PATENTSCHRIFT

(11) **EP 1 187 670 B1**
(45) Veröffentlichungstag und Bekanntmachung des Hinweises auf die Patenterteilung: **19.12.2007**
(21) Anmeldenummer: 00943741.9
(22) Anmeldetag: 26.05.2000
(51) Int. Cl.: B01D 53/94, F01N 11/00, F02D 41/02, G01N 27/417

(54) **KALIBRIERUNG EINES NOx-SENSORS UND REGELUNG EINES VORGESCHALTETEN NOx-SPEICHERKATALYSATORS**
CALIBRATION OF AN NOx SENSOR AND REGULATION OF AN UPSTREAM NOx STORAGE CATALYST
ETALONNAGE D'UN DETECTEUR DE NOx ET REGULATION D'UN CATALYSEUR ACCUMULATEUR DE NOx PLACE EN AMONT

(30) Priorität: 09.06.1999 DE 19926139
(43) Veröffentlichungstag der Anmeldung: 20.03.2002
(73) Patentinhaber: Volkswagen Aktiengesellschaft, 38436 Wolfsburg (DE)
(72) Erfinder: HAHN, Hermann, D-38165 Lehre (DE); HINZE, Sören, D-38114 Braunschweig (DE)
(86) Internationale Anmeldenummer: PCT/EP2000/004809
(87) Internationale Veröffentlichungsnummer: WO 2000/076636

(56) Entgegenhaltungen:
- EP-A- 0 814 248
- EP-A- 0 878 709
- EP-A- 0 916 941
- EP-A- 0 969 194
- US-A- 5 524 472
- PATENT ABSTRACTS OF JAPAN vol. 1995, no. 11, 26. Dezember 1995 (1995-12-26) & JP 07 208151 A (TOYOTA MOTOR CORP), 8. August 1995 (1995-08-08) & DATABASE WPI Section PQ, Week 199542 Derwent Publications Ltd., London, GB; Class Q51, AN 1995-323344
- PATENT ABSTRACTS OF JAPAN vol. 2000, no. 01, 31. Januar 2000 (2000-01-31) & JP 11 294224 A (NISSAN MOTOR CO LTD), 26. Oktober 1999 (1999-10-26)

## Beschreibung

Das Verfahren bezieht sich auf die Bestimmung des NOx-Gehalts des Abgases mittels eines NOx-Sensors und betrifft insbesondere ein Verfahren zum Kalibrieren eines derartigen NOx-Sensors sowie zum Steuern und Regeln eines NOx-Speicherkatalysators.

NOx-Sensoren zur Bestimmung des NOx-Gehalts im Abgas einer zumindest zeitweise magerlaufenden Brennkraftmaschine sind in verschiedenartiger Form bekannt und bedürfen keiner näheren Erläuterung. Derartig magerlauffähige Brennkraftmaschinen weisen in ihrem Abgastrakt üblicherweise einen NOx-Speicherkatalysator sowie zur Bestimmung des NOx-Gehalts des Abgases einen NOx-Sensor auf. Zur Einhaltung der NOx-Grenzwerte bei einem magerlauffähigen Verbrennungsmotor ist die Kenntnis des NOx-Gehalts im Abgastrakt hinter dem NOx-Speicherkatalysator notwendig, um den Zeitpunkt bestimmen zu können, an dem in dem NOx-Speicher keine Einlagerung von NOx mehr möglich ist, d.h. der NOx-Speicher gefüllt ist, so daß eine Regenerationsphase des Speichers notwendig ist. Das System verfügt ferner über eine Motorsteuerungseinheit, die unter anderem das gemessene NOx-Signal verarbeiten kann.

Die Anzeigegenauigkeit heute erhältlicher NOx-Sensoren weist ein unterschiedliches Fehlermaß vom tatsächlichen IST-Wert der NOx-Konzentration auf. Diese individuellen Ungenauigkeiten der Kennlinie des verwendeten Sensors können zu Fehlern im Steuerverhalten der Motorsteuereinheit führen. Falls beispielsweise aufgrund der Sensorungenauigkeit der Zeitpunkt der vollständigen Füllung des NOx-Speichers zu spät erkannt wird, wird die Regeneration zu spät eingeschaltet, was zu einer erhöhten Umweltbelastung führt.

Zur teilweisen Kompensation zumindest der Offsetungenauigkeit kann mindestens ein Betriebspunkt des Motors herangezogen werden, bei dem die NOx-Konzentration nach dem Katalysator mit hinreichender Genauigkeit angenommen werden kann. Das vom Sensor gemessene NOx-Signal wird dann mit Hilfe der NOx-Konzentration dieses Betriebspunktes korrigiert.

So kann für den NOx-Speicherkatalysator der folgende Betriebspunkt verwendet werden: In einem gewissen Temperaturbereich, beispielsweise bei ca. 350°C, kann selbst bei einem stark geschädigten oder vergifteten Katalysator zumindest für eine gewisse Zeit, belspielsweise ungefähr 5-10 Sekunden, nach einer vorangegangenen NOx-Regeneration im Leerlauf des Motors aufgrund des niedrigen NOx-Massenstroms von einer nahezu 100%-tigen NOx-Einlagerung ausgegangen werden. In diesem Fall beträgt der IST-Wert der NOx-Konzentration zwischen O und 5 ppm und dieser Wert des Betriebspunkts kann zur Korrektur des NOx-Sensors verwendet werden.

Ein derartiges Verfahren ist in der Anmeldung DE-199 11 664 beschrieben.

Allerdings besteht weiterhin bei der Regelung eines NOx-Speicherkatalysators das Problem, ob die im Leerlauf gewonnene Korrekturgröße im gesamten Bereich des zu regelnden Betriebs gültig ist. Ferner ist eine zeitliche Korrelation zwischen der Sensormessung und dem Betriebszustand des Motors, vorzugsweise eines Dieselmotors, nur bedingt möglich, da die Abgaslaufzeit und die Abgaslauflänge durch den NOx-Speicherkatalysator und die Trägheit des Sensors nur schwer berücksichtigt werden können.

EP 0 916 941 A2 betrifft ein Verfahren zur Bestimmung des Zustands eines NOx-Speicherkatalysators, in dem die NOx-Konzentration eines Abgasstroms mittels einen nach einem NOx-Speicher angeordneten Sensors bestimmt wird und wurde zur Bildung des Oberbegriffs des Anspruchs 1 herangezogen.

EP 0 878 709 A2 beschreibt ein Verfahren zur Bestimmung der NOx-Konzentration in einem Abgasstrom einer Brennkraftmaschine und einen entsprechenden Sensor.

Der Erfindung liegt daher die Aufgabe zugrunde, ein Verfahren zur Verbesserung der Korrekturvorgaben des NOx-Sensors sowie zur Steuerung eines NOx-Speicherkatalysators zu schaffen. Ferner soll eine entsprechende Vorrichtung geschaffen werden.

Diese Aufgabe wird durch die Merkmal des Verfahrens nach Anspruch 1 sowie der Vorrichtung nach Anspruch 3 gelöst. Bevorzugte Ausgestaltungen der Erfindung sind Gegenstand der Unteransprüche.

Eine bevorzugte Ausführungsform der Erfindung wird nachfolgend anhand der Zeichnungen erläutert.
- Fig.1: zeigt eine schematische Darstellung einer magerlauffähigen Brennkraftmaschine mit Abgasanlage,
- Fig. 2: zeigt die schematische Darstellung einer Sensorkennlinie, und
- Fig. 3: eine schematische Darstellung des Sensorsignals als Funktion der Zeit während des Umschaltens zwischen Regeneration- und Magerbetrieb.

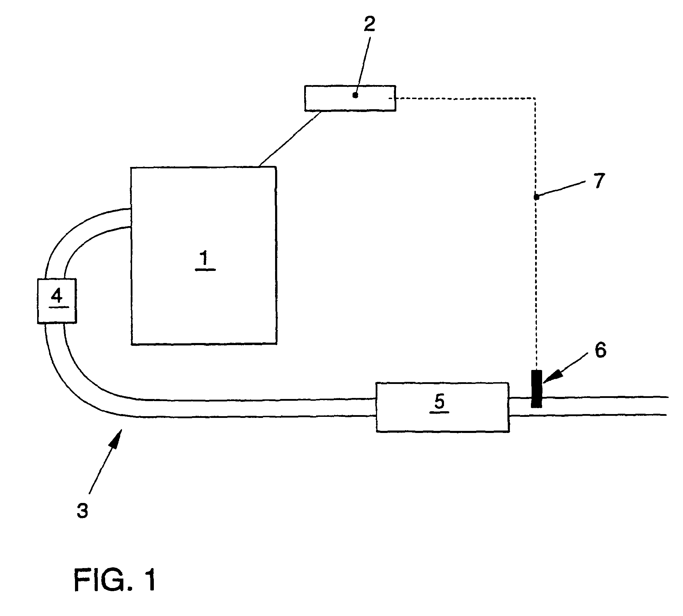

Fig. 1 zeigt schematisch ein magerlauffähiges motorisches System. Eine mageriauffähiger Brennkraftmaschine 1, die von einem Motormanagement 2 gesteuert wird, weist in seinem Abgastrakt 3 einen optionalen Vorkatalysator 4 sowie einen Katalysator mit NOx-Speicherfunktion 5 auf. In Abgasströmungsrichtung hinter dem Katalysator 5 ist ein NOx- oder Lambda-Sensor 6 angeordnet, der die NOx-Konzentration der Abgases mißt und sein Meßsignal über eine Leitung 7 an das Motormanagement 2 abgibt.

Wie bereits oben erwähnt, weisen heute erhältliche NOx-Sensoren 6 unterschiedliche Kennlinien auf, wobei je nach Hersteller ein unterschiedliches Fehlermaß vom tatsächlichen IST-Wert der NOx-Konzentration gegeben ist. Diese Ungenauigkeiten der Kennlinie des Sensors 6 haben, zumindest im linearen Bereich, mehrere Ursachen, und können unterteilt werden in die Genauigkeit, die den Offset 8 betrifft, und die Genauigkeit, die die Steigung 9 (Empfindlichkeit) betrifft.

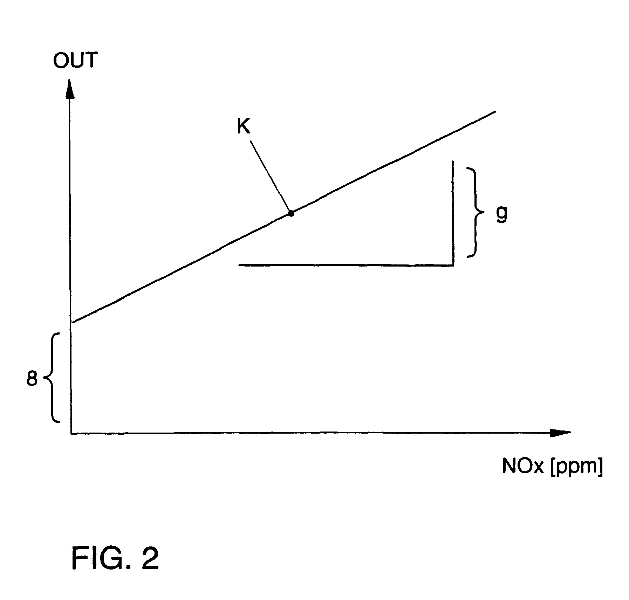

Fig. 2 zeigt eine Kennlinie K eines NOx-Sensors. Dargestellt ist das Sensorausgangssignal OUT in beliebigen Einheiten als Funktion der NOx-Konzentration NOx in ppm. Die in dem interessierenden Bereich lineare Kennlinie K weist einen Offset 8 (Achsenabschnitt) sowie eine Steigung 9 auf.

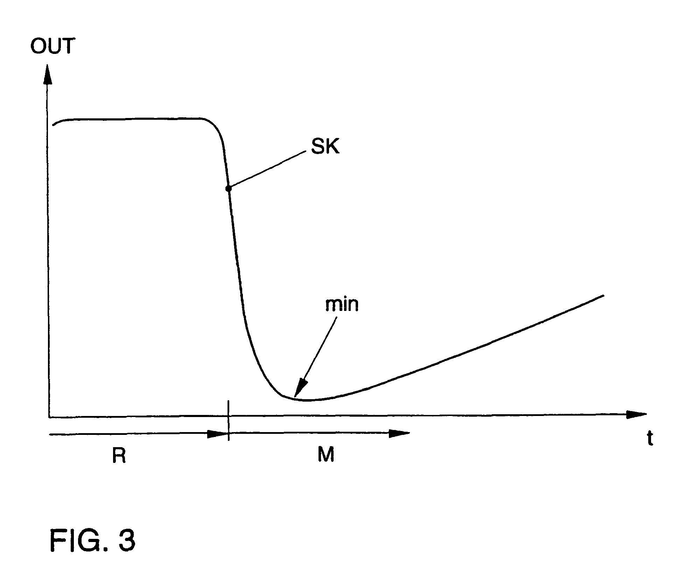

Fig. 3 zeigt schematisch in Form einer Sensorsignalkurve SK den Verlauf des NOx-Signal OUT als Funktion der Zeit t, wobei eine einer Regenerationsphase R nachfolgende Magerbetriebsphase M dargestellt ist. Grundsätzlich kommt es bei jedem NOx-Regenerationsvorgang R in einem NOx-Speicherkatalysator zu einem NOx-Desorptionspeak. Ferner wird NH₃ im Katalysator mit zunehmender Dauer der Regeneration gebildet. Beides führt zu einem Anstieg im NOx-Sensorsignal. Wird der Motor wieder mager betrieben (Magerphase M in Fig. 3), so speichert der Katalysator wieder NOx ein, das Sensorsignal OUT fällt, um danach mit zunehmender Beladung des Speicherkatalysators und dadurch zunehmender Tendenz zu NOx-Durchbrüchen wieder anzusteigen. Da jedoch die Abgaslauflänge und die Trägheit des Sensors nur schwer berücksichtigt werden können, ist eine zeitliche Korrelation der Sensormessung zu dem Motorbetriebszustand nur bedingt möglich.

Daher wird die Talsohle, d.h. das Minimum min der Sensorsignalkurve SK, den das Sensorsignal OUT während dieses Vorgangs erreicht, als Nullpunkt für den laufenden Speichervorgang angenommen und verwendet, insbesondere als zeitlicher Nullpunkt zur Steuerung des NOx-Speicherkatalysators. Zum Beispiel wird eine Auswertung des Sensorsignals, die beispielsweise zur Anforderung einer Katalysatorregeneration führen kann, erst ab diesem Zeitpunkt vorgenommen. Ferner kann für dieses Minimum min eine NOx-Konzentration von 0 angenommen werden, da von einer überwiegenden Einspeicherung der Stickoxide in den ersten Sekunden des Magerbetriebs ausgegangen wird. Daher wird der Wert dann auch als Betriebspunkt oder Referenzwert zur Bestimmung des Offsets des NOx-Sensors verwendet. Kommt es jedoch vor der Bestimmung dieses Nullpunkts , beispielsweise vor Ablauf einer vorbestimmten notwendigen Zeitspanne wie beispielsweise 10 Sekunden, zu Vorgängen, die die Kontinuität der Einspeicherung der Stickoxide in den NOx-Speicherkatalysator stören, so wird für diesen Einspeichervorgang nicht der aktuell bestimmte Nullpunkt als Referenz verwendet, sondern es wird ein vorbestimmter Referenzwert verwendet, der beispielsweise in dem verwendeten Kennfeld vorgegeben ist. Derartige Vorgänge, die die Kontinuität der Einspeicherung stören können, sind beispielsweise Schubabschaltungsphasen, nicht magerer Motorbetrieb oder starke Änderungen in der Motorlast.

### BEZUGSZEICHENLISTE

- 1: Magerlauffähige Brennkraftmaschine
- 2: Motormanagement
- 3: Abgasanlage
- 4: Vorkatalysator
- 5: Katalysator mit NOx-Speicherfunktion
- 6: NOx-/Lambda-Sensor
- 7: Verbindung
- 8: Offset
- 9: Steigung
- K: Kennlinie
- OUT: Ausgangssignal
- NOx[ppm]: NOx-Konzentration in ppm
- M: Magerphase
- R: Regenerationsphase
- min: Minimum
- t: Zeit
- SK: Sensorsignalkurve

## Patentansprüche

1. Verfahren zur Steuerung der Regelung eines NOx-Speicherkatalysators im Abgasstrom einer Brennkraftmaschine (1) mittels eines NOx-Sensors (6), wobei der Sensor (6) nach dem NOx-Speicherkatalysator (5) angeordnet ist und mittels mindestens einem ausgewählten Betriebspunkt kalibriert wird, wobei das Minimum (min) der Sensorsignalkurve (SK) nach einer Regenerationsphase (R) des Speicherkatalysators (5) als zeitlicher Nullpunkt zur Steuerung der Regelung des NOx-Speicherkatalysators und das Minimum (min) als Referenzwert zur Kalibrierung des Offsets (8) der Kennlinie des NOx-Sensors verwendet wird, **dadurch gekennzeichnet, dass** eine Übernahme des Nullpunkts (min) als Referenzwert unterbleibt, wenn es in zeitlicher Richtung vor der Bestimmung des Minimums innerhalb einer vorbestimmten Zeitspanne zu Vorgängen kommt, die die Kontinuität der Einlagerung des NOx stören und als Referenzwert vorübergehend ein vorbestimmter Referenzwert, beispielsweise aus einem Motorkennfeld, verwendet wird.

2. Verfahren nach Anspruch 1, **dadurch gekennzeichnet, daß** derartige Vorgänge durch eine Schubabschaltungsphase, ein stöchiometrischer oder fetter Motorbetrieb oder eine starke Änderung der Motorlast gebildet werden.

3. Brennkraftmaschine (1) mit einem sich im Abgastrakt (3) befindlichen NOx-Speicherkatalysator (5) sowie einem nachgeschalteten NOx-Sensor (6) und einer Motorsteuerungseinheit (2), wobei die Motorsteuereinheit (2) mit dem Verfahren nach einem der vorangegangenen Ansprüche den NOx-Speicherkatalysator (5) betreibt und den NOx-Sensor kalibriert, wobei die Brennkraftmaschine magerlauffähig ist.

## Claims

1. Method for controlling the regulation of a NOx storage catalytic converter in the exhaust gas flow of an internal combustion engine (1) by means of a NOx sensor (6), with the sensor (6) being arranged downstream of the NOx storage catalytic converter (5) and being calibrated by means of at least one selected operating point, with the minimum (min) of the sensor signal curve (SK) after a regeneration phase (R) of the storage catalytic converter (5) being used as a zero point in time for controlling the regulation of the NOx storage catalytic converter, and the minimum (min) being used as a reference value for calibrating the offset (8) of the characteristic curve of the NOx sensor, **characterized in that** the zero point (min) does not take over as the reference value if, within a predetermined timespan in the time direction before the determination of the minimum, processes occur which disturb the continuity of the storage of the NOx, and a predetermined reference value, for example from an engine characteristic map, is temporarily used as the reference value.

2. Method according to Claim 1, **characterized in that** such processes are formed by an overrun shut-off phase, a stoichiometric or rich engine operating mode, or an intense change in the engine load.

3. Internal combustion engine (1) having a NOx storage catalytic converter (5) which is situated in the exhaust tract (3) and having a NOx sensor (6) which is connected downstream, and having an engine control unit (2), with the engine control unit (2) operating the NOx storage catalytic converter (5), and calibrating the NOx sensor, using the method according to one of the preceding claims, with the internal combustion engine being capable of running in lean-burn mode.

## Revendications

1. Procédé de commande de la régulation d'un catalyseur accumulateur de NOx dans le flux des gaz d'échappement d'un moteur à combustion interne (1) au moyen d'un capteur de NOx (6), le capteur (6) étant disposé en aval du catalyseur accumulateur de NOx (5) et étant calibré au moyen d'au moins un point de fonctionnement dynamique, le minimum (min) de la courbe de signal du capteur (SK) après une phase de régénération (R) du catalyseur accumulateur (5) étant utilisé en tant que position zéro temporaire pour la commande de la régulation du catalyseur accumulateur de NOx et le minimum (min) étant utilisé en tant que valeur de référence pour le calibrage de l'offset (8) de la courbe caractéristique du capteur de NOx, **caractérisé en ce qu'**il n'est effectué aucune reprise de la position zéro (min) en tant que valeur de référence si, en direction temporaire, avant la détermination du minimum, on assiste au sein d'une période prédéfinie à des processus perturbant la continuité du stockage du NOx et **en ce qu'**une valeur de référence prédéfinie, issue par exemple d'un diagramme caractéristique du moteur est utilisée provisoirement en tant que valeur de référence.

2. Procédé selon la revendication 1, **caractérisé en ce que** des processus de ce type consistent en une phase de coupure d'alimentation en poussée, en un régime moteur stoechiométrique ou riche ou en une forte modification de la charge du moteur.

3. Moteur à combustion interne (1) avec un catalyseur accumulateur de NOx (5) se trouvant dans la ligne des gaz d'échappement (3), ainsi qu'avec un capteur de NOx (6) monté en aval et avec une unité de commande du moteur (2), l'unité de commande du moteur (2) faisant fonctionner le catalyseur accumulateur de NOx (5) et calibrant le capteur de NOx avec un procédé selon l'une quelconque des revendications précédentes, le moteur à combustion interne étant susceptible de fonctionner en régime pauvre.
